# EUROPEAN PATENT APPLICATION

(11) **EP 3 692 833 A1**
(43) Date of publication of application: **12.08.2020**
(21) Application number: 19155864.2
(22) Date of filing: 07.02.2019
(51) Int. Cl.: A24F 47/00, A61M 15/06, A61M 11/04

(54) **SMOKING SUBSTITUTE DEVICE**

(71) Applicant: Nerudia Ltd., Liverpool, Merseyside L24 9HP (GB)
(72) Inventor: The designation of the inventor has not yet been filed
(74) Representative: Mewburn Ellis LLP

(57) **Abstract**

A smoking substitute system (101) comprising a smoking substitute device (103) and a flavoured article (130). The smoking substitute device (103) has a fluid inlet (107), a fluid outlet (108), and an aerosol generator arranged between the inlet (107) and the outlet (108) and in fluid communication with both the inlet (107) and the outlet (108). The aerosol generator is operable to generate an aerosol from an aerosol precursor (104). The flavoured article (130) comprises a substrate (131) carrying a flavourant (132). The flavoured article (130) is arranged externally of the device (103) such that at least a part of the substrate (131) extends at least partially across the inlet (107) of the device (103) such that fluid drawn into the device (103) via the inlet (107) passes through the flavoured article (130) to thereby release flavourant (132) from the substrate (131) for entrainment in fluid entering the device (103) through the fluid inlet (107). The flavourant (132) comprises one or more substances effective to activate at least one of an olfactory receptor in a human nasal cavity and a taste receptor in a human oral cavity.

## Description

### Field of the Invention

The present invention relates to a smoking substitute device and, in particular, a smoking substitute device that is able to deliver flavour to a user.

### Background

The smoking of tobacco is generally considered to expose a smoker to potentially harmful substances. It is generally thought that a significant amount of the potentially harmful substances are generated through the heat caused by the burning and/or combustion of the tobacco and the constituents of the burnt tobacco in the tobacco smoke itself.

Combustion of organic material such as tobacco is known to produce tar and other potentially harmful byproducts. There have been proposed various smoking substitute systems in order to avoid the smoking of tobacco.

Such smoking substitute systems can form part of nicotine replacement therapies aimed at people who wish to stop smoking and overcome a dependence on nicotine.

Smoking substitute systems include electronic systems that permit a user to simulate the act of smoking by producing an aerosol (also referred to as a "vapour") that is drawn into the lungs through the mouth (inhaled) and then exhaled. The inhaled aerosol typically bears nicotine and/or a flavourant without, or with fewer of, the odour and health risks associated with traditional smoking.

In general, smoking substitute systems are intended to provide a substitute for the rituals of smoking, whilst providing the user with a similar experience and satisfaction to those experienced with traditional smoking and with combustible tobacco products.

The popularity and use of smoking substitute systems has grown rapidly in the past few years. Although originally marketed as an aid to assist habitual smokers wishing to quit tobacco smoking, consumers are increasingly viewing smoking substitute systems as desirable lifestyle accessories. There are a number of different categories of smoking substitute systems, each utilising a different smoking substitute approach.

One approach is the so-called "vaping" approach, in which a vaporisable liquid, typically referred to (and referred to herein) as "e-liquid", is heated by a heating device (referred to herein as an electronic cigarette or "e-cigarette" device) to produce an aerosol vapour which is inhaled by a user. The e-liquid typically includes a base liquid as well as nicotine and/or a flavourant. The resulting vapour therefore also typically contains nicotine and/or a flavourant. The base liquid may include propylene glycol and/or vegetable glycerine.

A typical e-cigarette device includes a mouthpiece, a power source (typically a battery), a tank for containing e-liquid, as well as a heating device. In use, electrical energy is supplied from the power source to the heating device, which heats the e-liquid to produce an aerosol (or "vapour") which is inhaled by a user through the mouthpiece.

E-cigarettes can be configured in a variety of ways. For example, there are "closed system" vaping smoking substitute systems, which typically have a sealed tank and heating element. The tank is prefilled with e-liquid and is not intended to be refilled by an end user. One subset of closed system vaping smoking substitute systems include a main body which includes the power source, wherein the main body is configured to be physically and electrically coupled to a consumable including the tank and the heating element. In this way, when the tank of a consumable has been emptied, that consumable is disposed of. The main body can be reused by connecting it to a new, replacement, consumable. Another subset of closed system vaping smoking substitute systems are completely disposable, and intended for one-use only.

There are also "open system" vaping smoking substitute systems which typically have a tank that is configured to be refilled by a user. In this way the entire device can be used multiple times.

An example vaping smoking substitute system is the myblu™ e-cigarette. The myblu™ e-cigarette is a closed system which includes a main body and a consumable. The main body and consumable are physically and electrically coupled together by pushing the consumable into the main body. The main body includes a rechargeable battery. The consumable includes a mouthpiece, a sealed tank which contains e-liquid, as well as a heater, which for this device is a heating filament coiled around a portion of a wick. The wick is partially immersed in the e-liquid, and conveys e-liquid from the tank to the heating filament. The device is activated when a microprocessor on board the main body detects a user inhaling through the mouthpiece. When the device is activated, electrical energy is supplied from the power source to the heating device, which heats e-liquid from the tank to produce a vapour which is inhaled by a user through the mouthpiece.

For a smoking substitute device it is desirable to deliver nicotine into the user's lungs, where it can be absorbed into the bloodstream. As explained above, in the so-called "vaping" approach, e-liquid is heated by a heating device to produce an aerosol vapour which is inhaled by a user. Many e-cigarettes also deliver flavour to the user to enhance the experience. In such e-cigarettes, flavour compounds are contained in the e-liquid that is heated. However, toxicology restrictions are placed on the amount of flavour that can be contained in the e-liquid, and this can result in some e-liquid flavours delivering a weak and underwhelming taste sensation to consumers in the pursuit of safety. Further, there is a view that providing a flavourant as part of the e-liquid, such that the flavourant is vaporised with the e-liquid, may be disadvantageous.

There may be a need for improved design of smoking substitute systems, in particular in regards to the delivery of flavour to a user.

The present disclosure has been devised in the light of the above considerations.

### Summary of the Invention

At its most general, the present invention relates to a flavoured article which can be placed over at least part of the fluid inlet of a smoking substitute system such that a flavourant may be provided to a user.

Providing a flavourant in this way may allow for increased versatility in the provision of flavour to the user of a smoking substitute system.

According to a first aspect of the present invention, there is provided a smoking substitute system comprising a smoking substitute device and a flavoured article, the smoking substitute device having a fluid inlet, a fluid outlet, and an aerosol generator arranged between the inlet and the outlet and in fluid communication with both the inlet and the outlet, the aerosol generator being operable to generate an aerosol from an aerosol precursor; wherein the flavoured article comprises a substrate carrying a flavourant, the flavoured article being arranged externally of the device such that at least a part of the substrate extends at least partially across the inlet of the device such that fluid drawn into the device via the inlet passes through the flavoured article to thereby release flavourant from the substrate for entrainment in fluid entering the device through the fluid inlet, the flavourant comprising one or more substances effective to activate at least one of an olfactory receptor in a human nasal cavity; and a taste receptor in a human oral cavity.

Optionally, the flavoured article is substantially annular and extends around at least part of the smoking substitute device.

Advantageously, at least part of the substrate has a porous structure.

Conveniently, at least part of the substrate is formed from an air-permeable material.

Optionally, at least part of the substrate is formed from a foamed material.

Advantageously, at least part of the substrate is formed from polymeric material.

Conveniently, the polymeric material is silicone.

Optionally, at least part of the substrate is formed from a deformable material.

Advantageously, at least part of the substrate is formed from a resiliently deformable material.

Conveniently, the deformable material is a stretchable material.

Optionally, the deformable material is a viscoelastic material.

Advantageously, the deformable material is an elastomeric material.

Conveniently, at least part of the substrate is substantially rigid.

Optionally, at least part of the substrate is impregnated with the flavourant.

Advantageously, at least part of the substrate is coated with the flavourant.

Conveniently, the flavoured article is a removable part of the smoking substitute system.

Optionally, the flavoured article is a replaceable part of the smoking substitute system.

Advantageously, the flavoured article is a consumable.

Conveniently, a part of the smoking substitute system comprising a source of aerosol precursor for the aerosol generator is a removable component of the smoking substitute system.

Optionally, said removable component is replaceable.

Advantageously, said replaceable component is a consumable.

Conveniently, the smoking substitute system includes a mouthpiece comprising said outlet.

Optionally, at least part of the flavoured article is arranged proximate to said mouthpiece.

Advantageously, the aerosol precursor is substantially free of flavourant.

Conveniently, the aerosol precursor is a liquid.

Optionally, the aerosol precursor contains nicotine.

According to a second aspect of the present invention, there is provided a flavoured article which is configured for use with the smoking substitute system according to the first aspect.

Optionally, a pack may be provided comprising a plurality of said flavoured articles.

The flavourant may include one or more volatile substances. The flavourant may be natural or synthetic. For example, the flavourant may include menthol, liquorice, chocolate, fruit flavour (including e.g. citrus, cherry etc.), vanilla, spice (e.g. ginger, cinnamon) and tobacco flavour. The flavourant may be evenly dispersed throughout the flavoured article, or may be provided in isolated locations and/or varying concentrations.

The smoking substitute apparatus (smoking substitute device) may be in the form of a consumable. The consumable may be configured for engagement with a main body (i.e. so as to form a closed smoking substitute system). For example, the consumable may comprise components of the system that are disposable, and the main body may comprise non-disposable or non-consumable components (e.g. power supply, controller, sensor, etc.) that facilitate the delivery of aerosol by the consumable. In such an embodiment, the aerosol precursor (e.g. e-liquid) may be replenished by replacing a used consumable with an unused consumable.

Alternatively, the smoking substitute device may be a non-consumable apparatus (e.g. that is in the form of an open smoking substitute system). In such embodiments an aerosol precursor (e.g. e-liquid) of the system may be replenished by re-filling e.g. a reservoir of the smoking substitute device with the aerosol precursor (rather than replacing a consumable component of the apparatus).

In light of this, it should be appreciated that some of the features described herein as being part of the smoking substitute device may alternatively form part of a main body for engagement with the smoking substitute device (i.e. when the smoking substitute device is in the form of a consumable).

Where the smoking substitute device is in the form of a consumable, the main body and the consumable may be configured to be physically coupled together. For example, the consumable may be at least partially received in a recess of the main body (or *vice-versa*), such that there is an interference fit between the main body and the consumable. Alternatively, the main body and the consumable may be physically coupled together by screwing one onto the other, or through a bayonet fitting.

Thus, the smoking substitute device may comprise one or more engagement portions for engaging with a main body. In this way, one end of the smoking substitute device may be coupled with the main body, whilst an opposing end of the smoking substitute device may define a mouthpiece of the smoking substitute system.

The smoking substitute device may comprise a reservoir configured to store an aerosol precursor, such as an e-liquid. The e-liquid may, for example, comprise a base liquid and e.g. nicotine. The base liquid may include propylene glycol and/or vegetable glycerine. The e-liquid may be flavourless. That is, the e-liquid may not contain any flavourants and may consist solely of a base liquid of propylene glycol and/or vegetable glycerine and nicotine.

The reservoir may be in the form of a tank. At least a portion of the tank may be translucent. For example, the tank may comprise a window to allow a user to visually assess the quantity of e-liquid in the tank. A housing of the smoking substitute device may comprise a corresponding aperture (or slot) or window that may be aligned with a translucent portion (e.g. window) of the tank. The reservoir may be referred to as a "clearomizer" if it includes a window, or a "cartomizer" if it does not.

The smoking substitute device may comprise a passage for fluid flow therethrough. The passage may extend through (at least a portion of) the smoking substitute device, between openings that define an inlet and an outlet of the passage. The outlet may be at a mouthpiece of the smoking substitute device. In this respect, a user may draw fluid (e.g. air) into and through the passage by inhaling at the outlet (i.e. using the mouthpiece). The passage may be at least partially defined by the tank. The tank may substantially (or fully) define the passage. In this respect, the tank may surround the passage.

The smoking substitute device may comprise an aerosol-generator. The aerosol generator may comprise a wick. The aerosol generator may further comprise a heater. The wick may comprise a porous material. A portion of the wick may be exposed to fluid flow in the passage. The wick may also comprise one or more portions in contact with liquid stored in the reservoir. For example, opposing ends of the wick may protrude into the reservoir and a central portion (between the ends) may extend across the passage so as to be exposed to fluid flow in the passage. Thus, fluid may be drawn (e.g. by capillary action) along the wick, from the reservoir to the exposed portion of the wick.

The heater may comprise a heating element, which may be in the form of a filament wound about the wick (e.g. the filament may extend helically about the wick). The filament may be wound about the exposed portion of the wick. The heating element may be electrically connected (or connectable) to a power source. Thus, in operation, the power source may supply electricity to (i.e. apply a voltage across) the heating element so as to heat the heating element. This may cause liquid stored in the wick (i.e. drawn from the tank) to be heated so as to form a vapour and become entrained in fluid flowing through the passage. This vapour may subsequently cool to form an aerosol in the passage.

The smoking substitute device (or main body engaged with the smoking substitute device) may comprise a power source. The power source may be electrically connected (or connectable) to a heater of the smoking substitute device (e.g. when engaged with the main body). The power source may be a battery (e.g. a rechargeable battery). A connector in the form of e.g. a USB port may be provided for recharging this battery.

When the smoking substitute device is in the form of a consumable, the smoking substitute device may comprise an electrical interface for interfacing with a corresponding electrical interface of the main body. One or both of the electrical interfaces may include one or more electrical contacts. Thus, when the main body is engaged with the consumable, the electrical interface may be configured to transfer electrical power from the power source to a heater of the consumable.

The electrical interface may also be used to identify the smoking substitute device (in the form of a consumable) from a list of known types. For example, the consumable may have a certain concentration of nicotine and the electrical interface may be used to identify this. The electrical interface may additionally or alternatively be used to identify when a consumable is connected to the main body.

Again, where the smoking substitute device is in the form of a consumable, the main body may comprise an interface, which may, for example, be in the form of an RFID reader, a barcode or QR code reader. This interface may be able to identify a characteristic (e.g. a type) of a consumable engaged with the main body. In this respect, the consumable may include any one or more of an RFID chip, a barcode or QR code, or memory within which is an identifier and which can be interrogated via the interface.

The smoking substitute device or main body may comprise a controller, which may include a microprocessor. The controller may be configured to control the supply of power from the power source to the heater of the smoking substitute device (e.g. via the electrical contacts). A memory may be provided and may be operatively connected to the controller. The memory may include non-volatile memory. The memory may include instructions which, when implemented, cause the controller to perform certain tasks or steps of a method.

The main body or smoking substitute device may comprise a wireless interface, which may be configured to communicate wirelessly with another device, for example a mobile device, e.g. via Bluetooth®. To this end, the wireless interface could include a Bluetooth® antenna. Other wireless communication interfaces, e.g. WiFi®, are also possible. The wireless interface may also be configured to communicate wirelessly with a remote server.

A puff sensor may be provided that is configured to detect a puff (i.e. inhalation from a user). The puff sensor may be operatively connected to the controller so as to be able to provide a signal to the controller that is indicative of a puff state (i.e. puffing or not puffing). The puff sensor may, for example, be in the form of a pressure sensor or an acoustic sensor. That is, the controller may control power supply to the heater of the consumable in response to a puff detection by the sensor. The control may be in the form of activation of the heater in response to a detected puff. That is, the smoking substitute device may be configured to be activated when a puff is detected by the puff sensor. When the smoking substitute device is in the form of a consumable, the puff sensor may form part of the consumable or the main body.

The invention includes the combination of the aspects and preferred features described except where such a combination is clearly impermissible or expressly avoided.

### Summary of the Figures

So that the invention may be understood, and so that further aspects and features thereof may be appreciated, embodiments illustrating the principles of the invention will now be discussed in further detail with reference to the accompanying figures, in which:
Figure 1 is a front view of an exemplary smoking substitute device and main body in an engaged position;
Figure 2 is a front view of the smoking substitute device and main body of Figure 1 in a disengaged position;
Figure 3 is a section view of an exemplary smoking substitute device;
Figure 4 is a front view of the exemplary smoking substitute device and main body illustrated in Figure 1 in combination with a flavoured article according to the present invention, the smoking substitute device being in an engaged position with the main body;
Figure 5 is a front view of the smoking substitute device and main body illustrated in Figure 4 in a disengaged position;
Figure 6 is a section view of the smoking substitute device illustrated in Figure 3 in combination with a flavoured article according to the present invention;
Figure 7 is an end on view of an exemplary flavoured article in accordance with the present invention, the flavoured article comprising a single flavourant;
Figure 8 is a section view of a part of a flavoured article in accordance with the present invention, wherein the flavourant is contained within pores in the substrate;
Figure 9 is a section view of a part of a flavoured article in accordance with the present invention, wherein the flavourant is coated on the substrate;
Figure 10 is an end on view of an exemplary flavoured article in accordance with the present invention, the flavoured article comprising two distinct flavourants;
Figure 11 is a section view of an exemplary smoking substitute system according to the present invention, and having a peripheral slot or groove for securing a flavoured article in place;
Figure 12 is a section view of the smoking substitute system illustrated in Figure 9, with the flavoured article in place;
Figure 13 is a section view of an exemplary smoking substitute system according to the present invention, and having a projecting lip or shoulder for securing a flavoured article in place;
Figure 14 is a section view of the smoking substitute system illustrated in Figure 11, with the flavoured article in place;
Figure 15 is a section view of an exemplary smoking substitute system according to the present invention, and having a clip or clasp for securing a flavoured article in place;
Figure 16 is a section view of the smoking substitute system illustrated in Figure 13, with the flavoured article in place;
Figure 17 is a section view of an exemplary smoking substitute system according to the present invention, and having a part of the outer casing extending across a peripheral groove or slot for securing a flavoured article in place; and
Figure 18 is a section view of the smoking substitute system illustrated in Figure 17, with the flavoured article in place.

### Detailed Description of the Invention

Aspects and embodiments of the present invention will now be discussed with reference to the accompanying figures. Further aspects and embodiments will be apparent to those skilled in the art. All documents mentioned in this text are incorporated herein by reference.

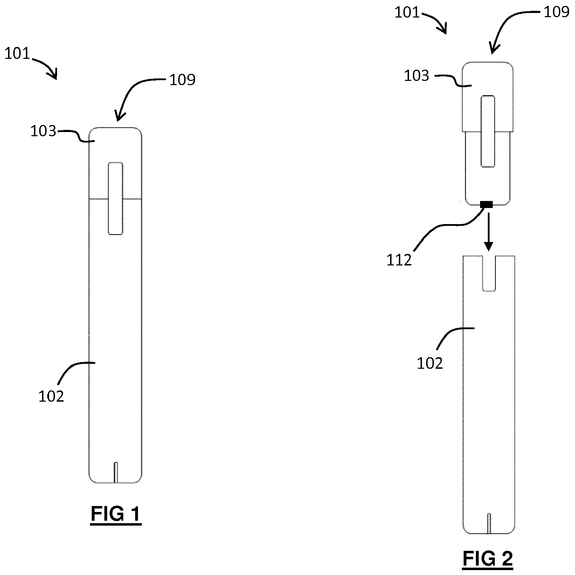

Figures 1 and 2 illustrate a smoking substitute system in the form of an e-cigarette system 101. The system 101 comprises an e-cigarette device defining a main body 102 of the system 101, and a smoking substitute device in the form of an e-cigarette consumable (or "pod") 103. In the illustrated embodiment the consumable 103 (smoking substitute device) is removable from the main body (e-cigarette device), so as to be a replaceable component of the system 101. In other words, the e-cigarette system 101 is a closed system.

As is apparent from Figures 1 and 2, the consumable 103 is configured to engage the main body 102. Figure 1 shows the main body 102 and the consumable 103 in an engaged state, whilst Figure 2 shows the main body 102 and the consumable 103 in a disengaged state. When engaged, a portion of the consumable 103 is received in a cavity of the main body 102 and is retained in the engaged position by way of a snap-engagement mechanism. In other embodiments, the main body 102 and consumable 103 may be engaged by screwing one into (or onto) the other, through a bayonet fitting, or by way of an interference fit.

The system 101 is configured to vaporise an aerosol former or aerosol precursor, which, in the illustrated embodiment, is in the form of a nicotine-based e-liquid 104. The e-liquid 104 comprises nicotine and a base liquid including propylene glycol and/or vegetable glycerine. In the present embodiment, the e-liquid 104 is flavourless and so does not include any added flavourant. That is, if the e-liquid 104 were to be inhaled (i.e. in aerosol form) by a user, it would not have a particularly perceptible flavour or taste. It is to be appreciated, however, that in other embodiments the e-liquid 104 may comprise an inherent flavourant. In the present specification, the term flavourant may be understood as referring to one or more substances effective to activate at least one of an olfactory receptor in a human nasal cavity; and a taste receptor in a human oral cavity.

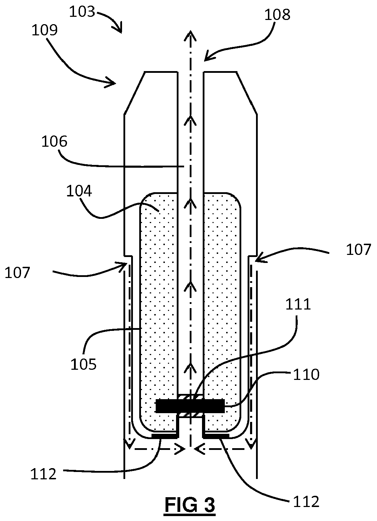

As is more apparent from Figure 3, the e-liquid 104 is stored within a reservoir in the form of a tank 105 that forms part of the consumable 103. In the illustrated embodiment, the consumable 103 is a "single-use" consumable 103. That is, upon exhausting the e-liquid 104 in the tank 105, the intention is that the user disposes of the entire consumable 103. In other embodiments, the e-liquid (i.e. aerosol precursor) may be the only part of the system that is truly "single-use". That is, the tank may be refillable with e-liquid or the e-liquid may be stored in a non-consumable component of the system. For example, the e-liquid may be stored in a tank located in the main body or stored in another component that is itself not single-use (e.g. a refillable cartomizer).

The tank 105 surrounds, and thus defines a portion of, a flow passage 106 that extends between an inlet 107 and an outlet 108 at opposing ends of the consumable 103. In this respect, the flow passage 106 comprises an upstream end at the end of the consumable 103 that engages with the main body 102, and a downstream end at an opposing end of the consumable 103 that comprises a mouthpiece 109 of the system 101. When the consumable 103 is engaged with the main body 102, a user can inhale (i.e. take a puff) via the mouthpiece 109 so as to draw air through the passage 106, and so as to form an airflow (indicated by arrows) in a direction from the inlet 107 to the outlet 108 of the passage 106. Although not illustrated, the passage 106 may be partially defined by a tube (e.g. a metal tube) extending through the consumable 103. The passage 106 is in fluid communication with a gap defined between the consumable 103 and the main body 102 (when engaged) such that air outside of the system 101 is drawn into the passage 106 (during an inhale).

The smoking substitute system 101 is configured to vaporise the e-liquid 104 for inhalation by a user. To provide this, the consumable 103 comprises a heater having of a porous wick 110 and a resistive heating element in the form of a heating filament 111 that is helically wound around a portion of the porous wick 110. The porous wick 110 and heating filament 111 may be referred to collectively as an aerosol generator. The aerosol generator is arranged between the inlet 107 and outlet 108 of the smoking substitute system 101, and is in fluid communication with both the inlet 107 and outlet 108. The porous wick 110 extends across the passage 106 (i.e. transverse to a longitudinal axis of the passage 106) and opposing ends of the wick 110 extend into the tank 105 (so as to be submerged in the e-liquid 104). In this way, e-liquid 104 contained in the tank 105 is conveyed from the opposing ends of the porous wick 110 to a central portion of the porous wick 110 so as to be exposed to the airflow in the passage 106 (i.e. caused by a user inhaling).

The helical filament 111 is wound about the exposed central portion of the porous wick 110 and is electrically connected to an electrical interface in the form of electrical contacts 112 mounted at the end of the consumable that is proximate the main body 102 (when engaged). When the consumable 103 is engaged with the main body 102, the electrical contacts 112 contact corresponding electrical contacts (not shown) of the main body 102. The main body electrical contacts are electrically connected to a power source (not shown) of the main body 102, such that (in the engaged position) the filament 111 is electrically connected to the power source. In this way, power can be supplied by the main body 102 to the filament 111 in order to heat the filament 111. This heat is transferred from the filament 111 to the porous wick 110 which causes e-liquid 104 conveyed by the porous wick 110 to increase in temperature to a point at which it vaporises. The vaporised e-liquid becomes entrained in the airflow. Between the vaporisation point at the filament 111 and the outlet 108 of the passage 106, the vaporised e-liquid condenses to form an aerosol. This aerosol is then inhaled via the mouthpiece 109 by a user of the system 101.

The power source of the main body 102 may be in the form of a battery (e.g. a rechargeable battery). The main body 102 may comprise a connector in the form of e.g. a USB port for recharging this battery. The main body 102 may also comprise a controller that controls the supply of power from the power source to the main body electrical contacts (and thus to the filament 111). That, is the controller may be configured to control a voltage applied across the main body electrical contacts, and thus the voltage applied across the filament 111. In this way, the filament 111 may only be heated under certain conditions (e.g. during a puff and/or only when the system is in an active state). In this respect, the main body 102 may include a puff sensor (not shown) that is configured to detect a puff (i.e. inhalation). The puff sensor may be operatively connected to the controller so as to be able to provide a signal, to the controller, which is indicative of a puff state (i.e. puffing or not puffing). The puff sensor may, for example, be in the form of a pressure sensor or an acoustic sensor.

Although not shown, the main body 102 and consumable 103 may comprise a further interface which may, for example, be in the form of an RFID reader, a barcode or QR code reader. This interface may be able to identify a characteristic (e.g. a type) of a consumable 103 engaged with the main body 102. In this respect, the consumable 103 may include any one or more of an RFID chip, a barcode or QR code, or memory within which is an identifier and which can be interrogated via the interface. A similar interface may also be comprised in the flavoured article 130 and smoking substitute device 103.

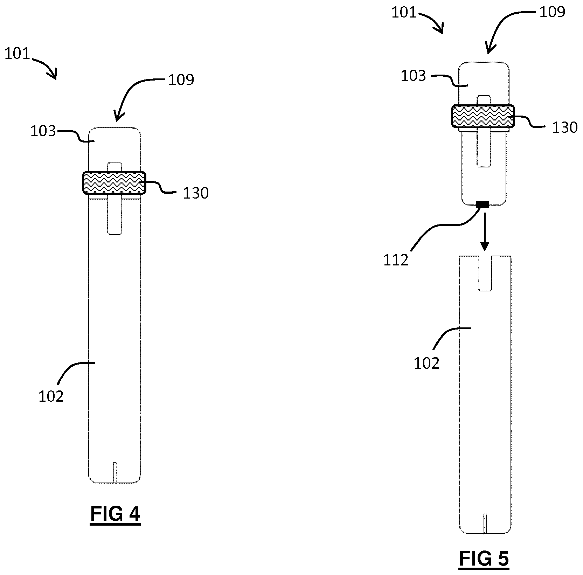

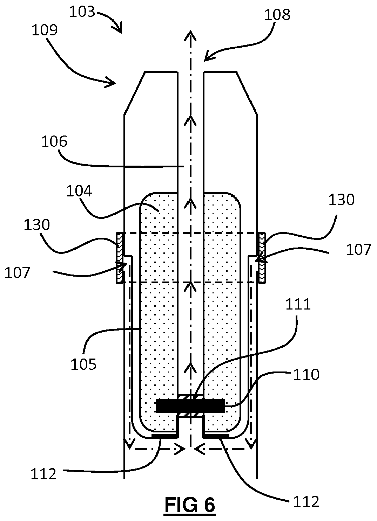

As is illustrated in Figures 4, 5, and 6, a flavoured article 130 is arranged external of the smoking substitute system such that it covers at least part of the inlet 107 of the smoking substitute system 101. Air drawn through the passage 106 by a user drawing via the outlet 108 or mouthpiece 109 therefore passes through the flavoured article 130. The flavoured article 130 includes a substrate 131 which carries a flavourant 132. Air drawn through the flavoured article 130 is effective to release flavourant 132 from the flavoured article 130 for entrainment in the airflow through the smoking substitute system 101.

For example, the flavourant 132 of the flavoured article 130 may have the same flavour as that in the e-liquid 104 in order to supplement the flavour provided within the e-liquid 104 and thereby provide the user with an intensified flavour sensation. Alternatively, the flavourant 132 of the flavoured article 130 may be complementary to that in the e-liquid 104 (i.e. the flavours/aromas of the two flavourants, when mixed, provide a pleasing sensory combination to a user). Providing the flavourant 132 separately from the aerosol precursor 104 affords the user an opportunity to select from or change between different flavourants 132 without necessitating a change of e-liquid, for example during a vaping session.

The flavoured article 130 may be arranged such that it extends towards the mouthpiece 109 from the inlet, along the exterior of the consumable pod 103, such that at least part of the flavoured article is arranged proximate to the mouthpiece 109 and will thus be proximate to the nose of a user during usage. Such an arrangement may allow an aroma from the flavoured article 130 to directly enter the nasal cavity of a user in addition to the flavourant entrained in the airflow passing through the smoking substitute device 103.

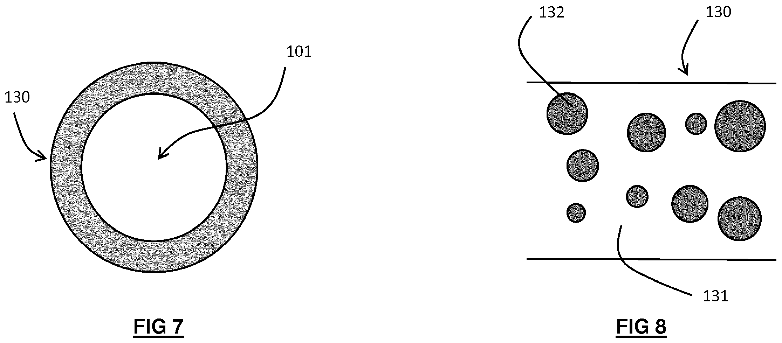

Figure 7 shows an exemplary flavoured article 130 according to the present invention. The flavoured article 130 is substantially annular in shape, such that it can extend around part of the smoking substitute device 101. While the flavoured article 130 is illustrated as substantially circular, it may be a different shape (e.g. elliptical, oval, rectangular) to correspond, at least approximately, with the shape of the exterior of smoking substitute device 103 proximate to its inlet 106. The flavoured article 130 may be removable from the smoking substitute device 103. A user may thereby select a flavoured article 130 which carries a different flavourant 132, allowing them to readily change flavour during a vaping session.

At least a part of the flavoured article 130 may be deformable or stretchable for ease of fitting and removal from the smoking substitute device 103. For example, at least a part the substrate 131 of the flavoured article may be formed from a deformable or stretchable material. It is to be appreciated that the natural shape of the flavoured article 130 (i.e. the shape of the flavoured article 130 when not fitted to the smoking substitute device 103) may be different to the external shape of the smoking substitute device 103. Such a flavoured article would be deformed by fitting to the smoking substitute device 103 such that it conforms to the external shape of said smoking substitute device 103.

At least a part of the flavoured article 130 may be resiliently deformable, such that it returns to or towards its original shape when an applied deforming force is removed. At least a part of the substrate 131 of the flavoured article may, for example, be formed from a resiliently deformable material. The resiliently deformable material from which said part of the substrate 131 is formed may, for example, be viscoelastic or elastomeric.

Alternatively or additionally, at least a part of the flavoured article 130 may be substantially rigid. For example, at least a part of the substrate 131 may be formed from a substantially rigid material.

At least part of the substrate 131 may be formed from a polymeric material (e.g. silicone). Further, at least part of the substrate 131 may be formed from a foam or foamed material. Still further, at least a part of the substrate 131 may be formed from an air permeable material.

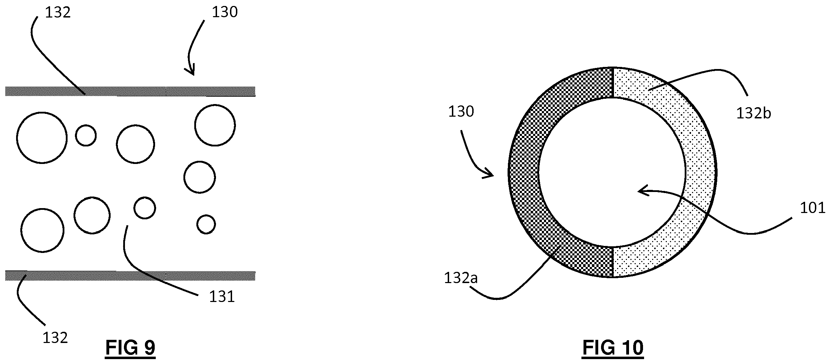

As illustrated in Figure 8, at least part of the substrate 131 may have a porous structure, and the pores may carry the flavourant 132. As illustrated in Figure 9, at least part of the substrate 131 may have a porous structure or be otherwise air permeable, but with flavourant 132 provided as a coating on the surface of the flavoured article 130. At least part of the flavoured article 130 may comprise the flavourant 132 both within the substrate 131, and as a coating on the surface of the substrate 131 (not illustrated). Flavourant 132 may be introduced into the flavoured article 130 by coating (e.g. spray coating). Alternatively or additionally, flavourant 132 may be introduced or impregnated into the flavoured article 130 by, for example, immersion of the substrate 131 in a liquid comprising flavourant 132.

The flavoured article 130 may be provided with a visual indication of the flavourant 132 therein. For example, at least part of the flavoured article 130 may be coloured according to the flavourant. Alternatively or additionally, the flavoured article 130 may be labelled with a textual or symbolic representation of the flavourant 132. Such a representation may be moulded in to the shape of the substrate 131, or printed on to the substrate 131. If a region of the flavoured article 130 is coloured, then at least part of the coloured region may be configured to "fade" (i.e. the colour becoming less intense) or otherwise change colour during use, to provide a visual indicator to the user of the amount of flavourant 132 remaining.

The flavoured article 130 may comprise a plurality of flavourants 132. Figure 10 illustrates an exemplary flavoured article comprising two flavourants 132a, 132b. For example, if the flavoured article 130 is annular, then regions comprising different flavourants 132 may form respective sectors of the annular shape (i.e. the regions may be arranged circumferentially, with each region being part of the circumference of the annular flavoured article 130). In such an embodiment, a user is able to rotate the flavoured article about the smoking substitute device 103 to place a different region over the inlet 107, and thereby select a different flavour. The parts of the flavoured article 130 with different flavourant 132 may be provided with a visual indication of the flavourant 132 therein (e.g. colour). The flavourants 132 comprised within adjacent regions may be selected to have complementary flavours/aromas. A user may thereby arrange the flavoured article 130 such that air drawn into the inlet 107 passes through both of two adjacent flavour regions to provide a mixture of flavours. Alternatively (not illustrated) a region substantially without flavourant may separate each of the regions comprising flavourant 132, ensuring that a user can receive a single desired flavourant 132, even from a flavoured article 130 comprising a plurality of flavourants 132a, 132b.

The flavoured article 130 may be attached to or fitted to the smoking substitute device 103 during manufacture of the smoking substitute device 103 or smoking substitute system 101. If the smoking substitute device 103 is in the form of a consumable, then the flavoured article 130 may be permanently attached to the smoking substitute device 103, such that the smoking substitute device 103 and flavoured article 130 collectively form a single consumable.

Alternatively, the flavoured article 130 may be removable from the smoking substitute device 103 or smoking substitute system 101, and may therefore itself be a consumable. In this case, the smoking substitute device may be shaped to ensure that the flavoured article 130 can be correctly positioned relative to the inlet 107.

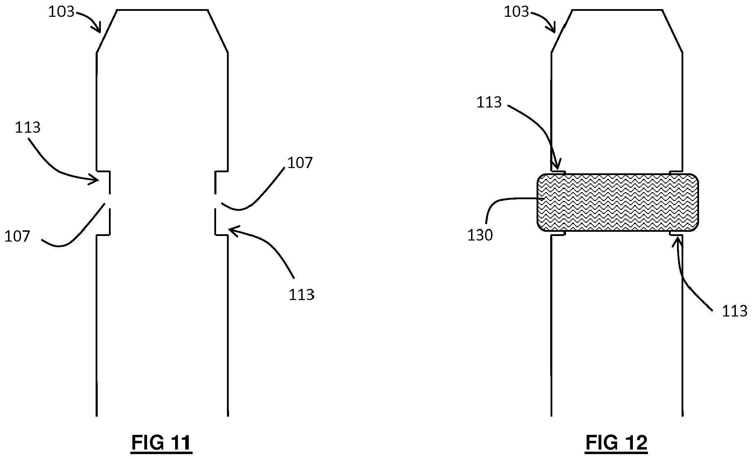

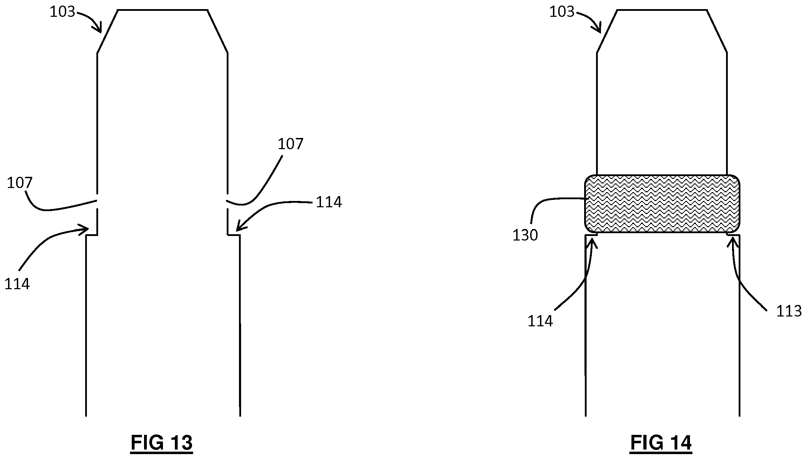

For example, as illustrated in Figures 11 and 12, the smoking substitute device 103 may comprise a peripheral groove, slot or recess 113 into which the flavoured article 130 can be fitted. As illustrated in Figures 13 and 14, the smoking substitute device 103 may have an outwardly projecting lip or shoulder 114 against which the flavoured article can be positioned. The slot 113 or lip 114 may be located at or in the region of the interface between the main body 102 and smoking substitute device 103. In this way, a user can fit the flavoured article 130 without having to move or slide it along the full length of the smoking substitute device 103, as they can instead disengage the main body 102 and 103 prior to fitting the flavoured article 130.

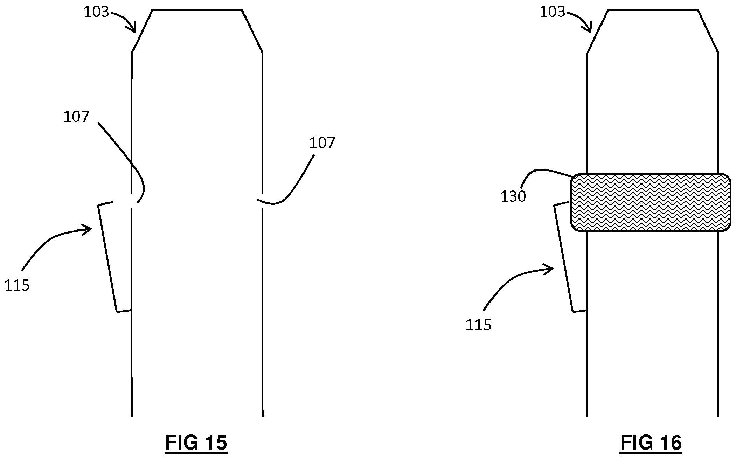

Alternatively or additionally, as illustrated in Figures 15 and 16, the smoking substitute system 101 may comprise a means of fastening (e.g. a clip or clasp) 115 by which the flavoured article 130 may be secured in place. Such a clip 115 may be fixed in position, allowing a flavoured article 130 to be fitted between it and the smoking substitute device 103. For example, the clip may be bendable to allow the flavoured article 130 to be fitted between it and the smoking substitute device 103. Alternatively, clip 115 may be hinged, for example at an attachment point between the clip 115 and the smoking substitute device 103, such that it can be angled towards or away from the smoking substitute device 103 to allow the flavoured article 130 to be fitted. Alternatively or additionally, the clip 115 may be attached to be rotatable about an axis perpendicular to the surface of the smoking substitute device 103. For example, the clip 115 may be attached to the smoking substitute device 103 by a bearing or a ball and socket joint. Alternatively, the clip 115 may be slideable along the length of the smoking substitute device 103, or around the exterior surface of the smoking substitute device 103. Any of the movable clips 115 described herein may further be spring-loaded to hold it in position. Further, any of the clips described herein may instead form part of the main body 102, and extend from there across the fitting position of the flavoured article 130.

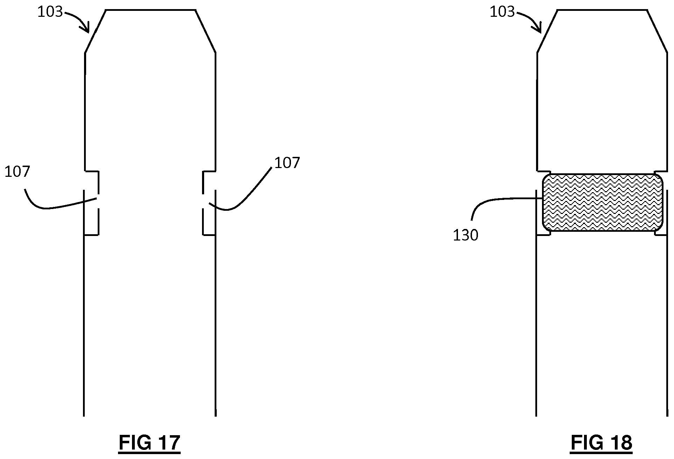

The flavoured article 130 may be fully located externally to the outer surface of the smoking substitute device 103, such as illustrated in the above-described embodiments. Alternatively, as illustrated in Figures 17 and 18, a part of the outer casing of the smoking substitute device 103 may be configured to cover at least a part of the flavoured article 130. This may, for example, further ensure that the flavoured article cannot be inadvertently moved once in place. The part of the exterior surface that covers the flavoured article 130 may, for example, be part of the main body 102 which is configured to overlap with a corresponding peripheral groove, slot, or recess in the smoking substitute device 103 when the main body 102 and smoking substitute device 103 are engaged. Alternatively, a part of the exterior surface of the smoking substitute device 103 may be configured as a slideable portion, which can then be moved to cover at least part of the flavoured article 130. Such a slideable portion may be spring-loaded to hold it in position.

Alternatively or additionally (not shown), the smoking substitute device 103 may include a marking (e.g. a line, arrow, or coloured region) to indicate correct positioning of the flavoured article 130.

A flavoured article 130, or a substitute smoking device 103 including a flavoured article 130 may be provided in a retail pack comprising one or more consumables. The pack may comprise consumables, each being or comprising a flavoured article 130 having the same flavourant 132. Alternatively, the pack may comprise consumables, each being or comprising a flavoured article 130 having a different flavourant 132 to provide a selection of possible flavourants to the user.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the words "have", "comprise", and "include", and variations such as "having", "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means, for example, +/- 10%.

The words "preferred" and "preferably" are used herein refer to embodiments of the invention that may provide certain benefits under some circumstances. It is to be appreciated, however, that other embodiments may also be preferred under the same or different circumstances. The recitation of one or more preferred embodiments therefore does not mean or imply that other embodiments are not useful, and is not intended to exclude other embodiments from the scope of the disclosure, or from the scope of the claims.

## Claims

1. A smoking substitute system (101) comprising:
a smoking substitute device (103) and a flavoured article (130);
the smoking substitute device (103) having a fluid inlet (107), a fluid outlet (108), and an aerosol generator arranged between the inlet (107) and the outlet (108) and in fluid communication with both the inlet (107) and the outlet (108), the aerosol generator being operable to generate an aerosol from an aerosol precursor (104); **characterized in that**
the flavoured article (130) comprises a substrate (131) carrying a flavourant (132), the flavoured article (130) being arranged externally of the device (103) such that at least a part of the substrate (131) extends at least partially across the inlet (107) of the device (103) such that fluid drawn into the device (103) via the inlet (107) passes through the flavoured article (130) to thereby release flavourant (132) from the substrate (131) for entrainment in fluid entering the device (103) through the fluid inlet (107),
the flavourant (132) comprising one or more substances effective to activate at least one of:
an olfactory receptor in a human nasal cavity; and
a taste receptor in a human oral cavity.

2. A smoking substitute system (101) according to claim 1, wherein
the flavoured article (130) is substantially annular and extends around at least part of the smoking substitute device (103).

3. A smoking substitute system (101) according to any one of claims 1 or 2, wherein
at least part of the substrate (131) has a porous structure.

4. A smoking substitute system (101) according to any one of the preceding claims, wherein
at least part of the substrate (131) is formed from an air-permeable material.

5. A smoking substitute system (101) according to any one of the preceding claims, wherein
at least part of the substrate (131) is formed from polymeric material.

6. A smoking substitute system (101) according to any one of the preceding claims, wherein
at least part of the substrate (131) is formed from a deformable material.

7. A smoking substitute system (101) according to any one of the preceding claims, wherein
at least part of the substrate (131) is formed from a resiliently deformable material.

8. A smoking substitute system (101) according to any one claims 6 - 7, wherein
the deformable material is a viscoelastic material.

9. A smoking substitute system (101) according to any one of the preceding claims, wherein
at least part of the substrate (131) is substantially rigid.

10. A smoking substitute system (101) according to any one of the preceding claims, wherein
at least part of the substrate (131) is impregnated with the flavourant (132).

11. A smoking substitute system (101) according to any one of the preceding claims, wherein
at least part of the substrate (131) is coated with the flavourant (132).

12. A smoking substitute system (101) according to any one of the preceding claims, wherein
the flavoured article (130) is a removable part of the smoking substitute system (101).

13. A smoking substitute system (101) according to any one of the preceding claims, having a mouthpiece (109) comprising said outlet (108), wherein at least part of the flavoured article (130) is arranged proximate to said mouthpiece (109).

14. A smoking substitute system (101) according to any one of the preceding claims, wherein
the aerosol precursor (104) is substantially free of flavourant.

15. A flavoured article (130) configured for use with the smoking substitute system (101) of any one of the preceding claims.
